# EUROPEAN PATENT SPECIFICATION

(11) **EP 1 244 402 B1**
(45) Date of publication and mention of the grant of the patent: **05.07.2006**
(21) Application number: 00930710.9
(22) Date of filing: 12.05.2000
(51) Int. Cl.: A61F 13/15, A61F 13/20

(54) **TAMPON WITH COVER AND NONIONIC SURFACTANT**
TAMPON MIT ÄUSSERER SCHICHT UND NICHT-IONISCHE OBERFLÄCHENAKTIVE SUBSTANZ
TAMPON HYGIENIQUE RECOUVERT D'UNE PELLICULE COMPRENANT UN TENSIOACTIF NON IONIQUE

(30) Priority: 30.06.1999 US 345088
(43) Date of publication of application: 02.10.2002
(73) Proprietor: McNEIL-PPC, INC., Skillman, NJ 08858 (US)
(72) Inventor: ANDOL, Joseph, R., Starkville, MS 39759 (US); GELL, Carol, B., Belle Mead, NJ 08502 (US); LOUIE, Lai-Hing, Kendall Park, NJ 08824 (US); PIERSON, Linda, M., Somerville, NJ 08876 (US); ROLLER, Judith, North Brunswick, NJ 08902 (US)
(74) Representative: Metten, Karl-Heinz
(86) International application number: PCT/US2000/013206
(87) International publication number: WO 2001/001905

(56) References cited:
- WO-A-95/23571
- WO-A-98/10726
- US-A- 3 683 912
- US-A- 5 342 334
- US-A- 5 647 862

## Description

### Cross Reference to Related Applications

This invention is related to the following copending applications: WO 01/02144 A1, entitled "Continuous Method of Providing Individual Sheets from a Continuous Web"; US 2003/0093049 A1, entitled "Multilayered Apertured Film Wrapping Element for Absorbent Articles"; WO 01/01909 A1, entitled "Domed Tampon with Surfactant-Treated Cover"; US 6,537,414 B1, entitled "Sealing Roller and Sealing Roller Element, Particularly for Producing a Tampon for Feminine Hygiene and Method Therefore"; and WO 01/01908 A1, entitled "Tampon for Feminine Hygiene and Process and Apparatus for its Production".

### Background of the Invention

The present invention relates to a tampon having an apertured film cover. The cover has a nonionic surfactant applied thereto. The nonionic surfactant provides improved fluid transfer across the cover, and it reduces frictional forces during processing of the tampon.

There are several types of covers that have been or are currently in use for tampons: woven fabrics, nonwoven fabrics, apertured films, reticulated films, polymer nets, and the like. While the patent literature has suggested that a progression from nonwoven fabrics to apertured films in these covers is desired, this has not yet occurred commercially. In order to commercialize this desirable tampon, several issues must be overcome. First, apertured films are generally hydrophobic in nature, and this can reduce the ease with which bodily fluids can be accepted into the absorbent structure enclosed within the covers. Second, apertured films have significantly different friction characteristics than nonwoven fabrics. This can create processing difficulties, especially in tampon presses in which compressed tampons are subjected to axial ejection forces, such as disclosed in US Pat. Nos. 3,343,225 (Hochstrasser et al.), 3,348,866 (Etz), 3,422,496 (Wolff et al.), 3,477,102 (Etz) 3,515,138 (Hochstrasser et al.), 3,688,346 (Johst et al.), 3,852,847 (Etz), 4,081,884 (Johst et al.), 4,498,218 (Friese), and 4,453,296 (Friese), European Pat. App. No. 0 623 333 (Karl Ruggli AG), European Pat. App. No. 0 639 363 (Karl Ruggli AG), and European Pat. No. 0 422 660 (Johnson & Johnson GmbH).

Nonwoven fabric covers may be incorporated into tampons as described in Friese, US Pat. Nos. 4,816,100; 4,836,450; and 4,859,273. These tampons can then be made into tampons as described in the patents identified above.

Apertured film covers have been incorporated into sanitary napkins to increase the products' ability to hide absorbed bodily fluids. An example of such an apertured film cover is disclosed in McNeil-PPC, Inc., EP 0 900 071. This advancement relates to the corona treatment of an apertured film for application thereon of a water-borne surfactant. This significant advance in the art, nonetheless requires numerous processing steps, and there sanitary napkins produced therefrom are not subjected to the high axial friction seen in the tampon manufacturing processes described above.

The WO 98/10726 relates to a process for forming a single-layer, durably wettable polymeric web having a plurality of apertures. The process comprises melting a mixture of at least one thermoplastic polymer and at least one surfactant and extruding said mixture to form a single-layer, substantially continuous polymeric film which is apertured by use of high pressure fluid flows. This apertured polymeric film should be suitable as a top sheet for absorbent articles. According to one embodiment pellets of the surfactant and one or more polymers can be first dry blended and then melt mixed in an extruder. In case an insufficient mixing occurs in the extruder, the pellets of the polymer and the surfactant can be first dry blended and then melt mixed in a pre-compounding extruder followed by repelletisation prior to film extrusion. Apertures are formed by use of a coronar discharge treatment. Accordingly, WO 98/10726 provides for fluid aperturing a continuous polymeric film wherein said process minimizes surfactant wash-off.

The US 5,647,862 discloses a sanitary napkin having a top sheet, a back sheet and an absorbent core. The absorbent article comprises a means for directional fluid distribution such as a fluid directing strip positioned between the top sheet and the absorbent core, and also an absorbent strip positioned between the top sheet and fluid directing strip. These strips are arranged such that bodily exudates may be directed toward the ends of the absorbent core even when the sanitary napkin assumes a body-conforming shape or is otherwise distorted during use. With the design according to US 5,647,862 D2 the problem should be solved that the entire absorbent capacity of the sanitary napkin is utilized before liquids reach the longitudinal side edges of said napkin.

US 3,683,912 discloses an absorbing tampon that has a fluid pervious layer of polypropylene fibers disposed on its outer surface. The non-woven polypropylene wrapper might also be treated with a wetting agent such as a non-ionic agent in order to speed up the penetration of fluids during use.

WO 95/23571 discloses a process for making a surfactant treated formed polymeric web which should be suited for use as a top sheet, and absorbent articles such as sanitary napkins, pantiliners, disposable diapers and incontinent articles. The polymeric web is obtained from a co-extruded multilayer polymeric film having a core layer and at least one outer layer wherein the core layer includes a surfactant (page 2, lines 21 to 24). The polymer and the surfactant are mixed in pellet form in a paddle mixer and then extruded to form a thorough mixture.

US 5,342,334 discloses an absorbent article including a fluid pervious top sheet which comprises a three-dimensional microscopically expanded fluid pervious web including a first polymeric material and a second polymeric material which have different melting point temperatures wherein the melting point of the second polymeric material is less than the melting point temperature of the first polymeric material. The web is heated to a temperature between the two melting point temperatures, so that the second polymeric material can be bonded to the intermediate layer along a second surface of the web.

Therefore, what is needed is a tampon having an apertured film cover that is processable in a commercially efficient manner.

### Summary of the Invention

The present invention relates to tampons according to claim 1 having an apertured film cover that are able to be manufactured in a commercially efficient manner, the tampons having an apertured film cover that has a nonionic surfactant coating, and to a process according to claim 15, including the nonionic surfactant to the cover that results in reduced ejection forces when the substantially cylindrical, compressed tampon is ejected from a tampon forming press in an axial direction.

Preferred embodiments of the tampons are in accordance with sub-claims 2 to 14, whereas preferred embodiments of the process for the manufacture of a tampon are in accordance with sub-claims 16 to 21. The tampon includes an absorbent structure substantially enclosed by a cover. The cover has fluid-impervious plastic material in the form of a resilient three-dimensional web exhibiting a fiber-like appearance and tactile impression. The web has first and second surfaces, and the first surface has a multiplicity of apertures therein. The fluid-impervious plastic material is coated with about 0.5 to about 2 wt-% of a nonionic surfactant.

The process for the manufacture of a tampon includes several steps. A nonionic surfactant is applied to a web of fluid-impervious plastic material. The web is in the form of a resilient three-dimensional web exhibiting a fiber-like appearance and tactile impression. The web has opposed first and second edges and a substantially infinite length and first and second surfaces. The first surface has a multiplicity of apertures therein.

An individual sheet is separated from the web and attached to an absorbent structure. The absorbent structure is formed into a tampon blank wherein the individual sheet substantially encloses the tampon blank. The tampon blank is compressed in a press to form a substantially cylindrical, compressed tampon having a cover comprising the individual sheet. The compressed tampon is ejected from the press by application of an axial ejection force. The application of the nonionic surfactant is sufficient to reduce the ejection force of the compressed tampon from the press to less than about 1100 N.

### Brief Description of the Drawing

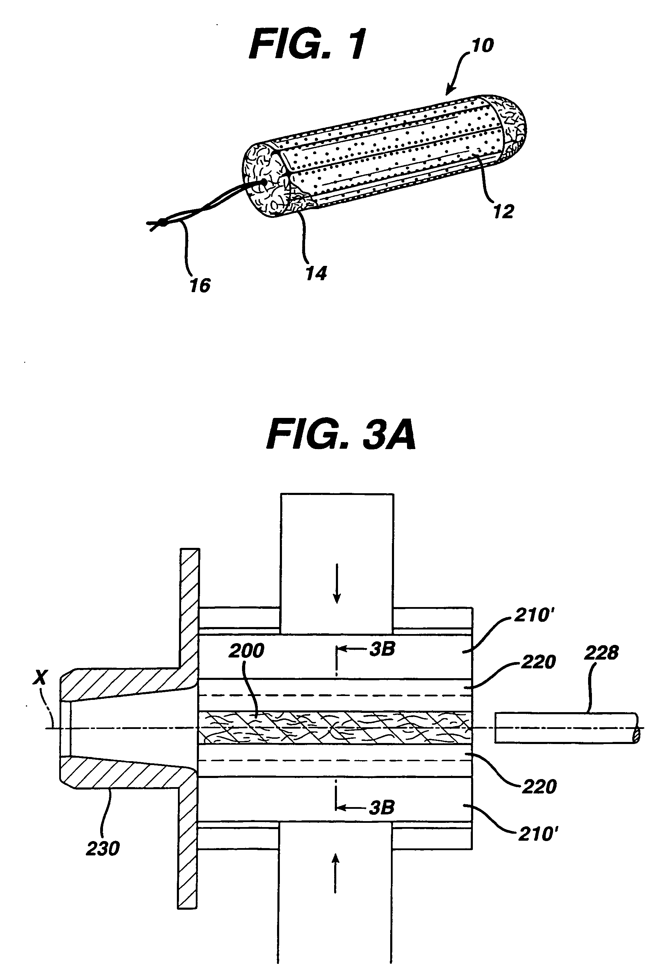

Fig. 1 is a perspective view of a tampon having an apertured film cover according to the present invention.
Figs. 2A-2C are cross-sectional views illustrating the sequence of producing a tampon having an apertured film cover according to the present invention.
Fig. 3A is a longitudinal cross section of an apparatus for producing a tampon having an apertured film cover according to the present invention.
Figs. 3B and C are cross-sectional views along line 3B-3B of Fig. 3A illustrating the sequence of producing a tampon having an apertured film cover according to the present invention.

### Detailed Description of the Preferred Embodiment

As used herein, the term "apertured film" refers to a fluid-impervious plastic material in the form of a resilient three-dimensional web having first and second surfaces and exhibiting a fiber-like appearance and tactile impression. The first surface of the three-dimensional web has a multiplicity of apertures therein. Preferably, each of the apertures is defined by a multiplicity of intersecting, fiber-like elements interconnected to one another substantially in the plane of the first surface. Each of the fiber-like elements exhibits a cross-section, preferably having a base portion in the plane of the first surface and a sidewall joined to each edge of the base portion. The sidewall portions extend generally in the direction of the second surface of the three-dimensional web. Further, the intersecting sidewall portions are interconnected to one another intermediate the first and second surfaces of the web. The interconnected sidewall portions preferably terminate substantially concurrently with one another in the plane of the second surface.

The tampon 10 of the present invention comprises an apertured film cover 12, and an absorbent structure 14. The cover at least partially encloses the absorbent structure 14 that is generally designed and constructed to absorb and contain bodily exudates. The tampon 10 also includes removal means, such as a withdrawal string 16.

The absorbent structure may be any absorbent means that is capable of absorbing and/or retaining liquids (e.g., menses and/or urine). The absorbent structure can be manufactured in a wide variety of sizes and shapes and from a wide variety of liquid-absorbing materials. A representative, non-limiting list of useful materials includes cellulosic materials, such as rayon, cotton, wood pulp, creped cellulose wadding, tissue wraps and laminates, peat moss, and chemically stiffened, modified, or cross-linked cellulosic fibers; synthetic materials, such as polyester fibers, polyolefin fibers, absorbent foams, absorbent sponges, superabsorbent polymers, absorbent gelling materials; formed fibers, such as capillary channel fibers and multilimbed fibers; combinations of materials, such as synthetic fibers and wood pulp including coformed fibrous structures (e.g., those materials described in Anderson et al., U.S. Patent No. 4,100,324); or any equivalent material or combinations of materials, or mixtures of these.

The removal means may be any element or device that is useful to remove the tampon from the bodily cavity after use. A representative, non-limiting list of useful removal means includes string, including spun fibers and monofilament line, tape, and the like. Preferable removal means include string.

The cover or the present invention is formed of a web having first surface and second surface. The web may be formed of a heterogeneous, monolayer film, or it may be formed of a laminate having a plurality of layers, such as is described in the commonly assigned, corresponding application US 2003/0093049 A 1 entitled "Multilayered Apertured Film Wrapping Element For Absorbent Articles".

The apertured film is formed of at least one blend of at least two immiscible polymeric materials. A representative, non-limiting list of polymeric materials that may be used in the apertured film includes polyolefins, such as polypropylene and polyethylene; polyolefin copolymers, such as ethylene-vinyl acetate ("EVA"), ethylene-propylene, ethylene-acrylates, and ethylene-acrylic acid and salts thereof; halogenated polymers; polyesters and polyester copolymers; polyamides and polyamide copolymers; polyurethanes and polyurethane copolymers; polystyrenes and polystyrene copolymers; and the like. Preferred polymeric materials include polyolefins, especially polyethylene and polypropylene and ethylene copolymers, especially EVA.

In a heterogeneous apertured film, either monolayer or multilayer, the film preferably includes a blend of at least two thermoplastic polymeric components. The first thermoplastic polymeric component forms a continuous phase that exhibits a first melting point temperature. In order to form the continuous phase, it is preferred that the first thermoplastic polymeric component be present at about 45 to about 95 wt-% of the layer, more preferably about 60 to about 80 wt-% of the layer. A dispersed phase comprises a second thermoplastic polymeric component that exhibits a second melting point temperature. It is preferred that the second thermoplastic polymeric component is present at about 55 to about 5 wt-% of the layer, more preferably about 80 to about 60 wt-% of the layer. In addition, the second melting point temperature is sufficiently less than the first melting point temperature to allow the film to be heated to a temperature between the first and second melting point temperatures, rendering the second thermoplastic polymeric component capable of forming an adhesive bond. This bond may be formed between different portions of the cover, or it may be between the cover and another element of the tampon. Preferably, the difference between the first melting point temperature and the second melting point temperature is greater than about 20° C, and more preferably, the difference between the first melting point temperature is greater than about 30° C. Most preferably, the difference is greater than about 40° C.

The continuous phase provides the "backbone" of the layer and contributes most of the layer's mechanical properties, such as tensile strength, stiffness, elongation at break, coefficient of friction, modulus, and the like. The first thermoplastic polymeric component substantially provides these properties. Therefore, the first thermoplastic polymeric component of the first layer will be chosen for its desirable properties. In contrast, the dispersed phase provides localized areas for thermal bonding to adjacent elements. The second thermoplastic polymeric component provides these properties, and it may also contribute to some of the mechanical properties, including coefficient of friction.

In addition, other components and further additives can be added to the polymeric material in an amount that will not hinder obtaining the object of the present invention, including, without limitation, antioxidants, UV absorbers, lubricants, antiblock and slip agents, plasticizers, nucleating agents, antistatic agents, flame retardants, pigments, dyes, and inorganic or organic fillers.

The nonionic surfactant is preferably an ethoxylate. In one embodiment of the inventive tampon said ethoxylate is an ethoxylated fatty acid polyolester. In a preferred embodiment said ethoxylate is an ethoxylated fatty acid sorbitan ester. A representative, non-limiting list of useful sorbitan esters includes polyoxyethylene sorbitan laurate (also known as Polysorbate 20 and 21), polyoxyethylene sorbitan palmitate (also known as Polysorbate 40), polyoxyethylene sorbitan stearate (also known as Polysorbate 60 and 61), polyoxyethylene sorbitan tristearate (also known as Polysorbate 65), polyoxyethylene sorbitan oleate (also known as Polysorbate 80 and 81), and polyoxyethylene sorbitan trioleate (also known as Polysorbate 85). Among the aforementioned ethoxylated fatty acid sorbitan esters, polyoxyethylene-20-sorbitan monolaurate is most preferred.

In an alternative embodiment of the inventive tampon, the ethoxylate is a polyoxyethylene alkyl ether. A representative, non-limiting list of useful polyoxyethylene alkyl ethers includes polyoxyethylene lauryl ether, polyoxyethylene stearyl ether (also known as Steareth-2, Steareth-10, and the like), polyoxyethylene cetyl ether (also known as Ceteth-2, Ceteth-10, and the like), and polyoxyethylene oleyl ether (also known as Oleth-2, oleth-10, and the like). Among the aforementioned polyoxyethylene alkyl ethers, polyoxyethylene stearyl ether is most preferred.

In a third alternative embodiment the ethoxylate is an ether of an olefinic diol. A representative, non-limiting list of such olefinic diols useful in the present invention includes the following: polyethylene glycol, polypropylene glycol, polybutylene glycol, propylene glycol, and the like. The olefinic diols are pfreferably liquid at a temperature of less than about 35° C. This weight is typically dictated by their molecular weight. As used herein in the specification and claims, the term "molecular weight" refers to the number average molecular weight of a compound. Preferably, the olefinic diol is polyethylene glycol, having a molecular weight of less than about 600, or polypropylene glycol, having a molecular weight of less than about 4,000. Most preferably, olefinic diol is polyethylene glycol, having an average molecular weight of less than about 600. Preferred esters and ethers of fatty acids and salts thereof include, without limitation, mono- and diesters of C₈₋₁₈ fatty acids and polyhydric alcohols, such as glyceryl monolaurate, applied neat from a melt or dissolved in polyethylene glycol as described in the copending application to Yang, entitled "Process for Adding Pharmaceutically Active Compounds to Substrates", US 6,316,019 B1.

It is to be understood that the nonionic surfactants used in the tampon and in its manufacture as described herein may be commercially available. Examples thereof are marketed under the registered trademarks "TWEEN" and "BRIJ" of ICI, Atlas Chemical Division, Wilmington, DE, USA.

The nonionic surfactant applied to the cover may serve at least two functions. First, the nonionic surfactant may render hydrophilic an apertured film that comprises generally hydrophobic polymeric materials. Second, the nonionic surfactant significantly reduces ejection forces when the tampon is ejected from a tampon forming press in an axial direction. Therefore, the nonionic surfactant has properties that improve the affinity of the apertured film for bodily fluids and reduces friction between the tampon and manufacturing equipment.

The nonionic surfactant is applied to the cover in an amount sufficient to reduce tampon press ejection forces sufficiently to prevent damage to the product during manufacture. Damage to the product includes destroying the product due to press jams in which the product stops the machine. Damage to the product also includes distortion of the cover due to friction between the cover and manufacturing equipment. This may be viewed as "shingling" of the product. Shingling of the product occurs when pleats perpendicular to the tampon's longitudinal axis are fomed. This may take the appearance of a series of rings around the products due to these circumferential pleats. Preferably, the nonionic surfactant is applied to the cover in an amount sufficient to reduce tampon press ejection forces to less than about 1100 N. More preferably, the tampon press ejection forces are reduced to less than about 1000 N.

These reduced ejection forces may be achieved by applying a coating weight of up to about 0.5 grams/meter² ("gsm"). More preferably, the coating weight is about 0.1 to about 0.4 gsm, and most preferably, it is applied at a coating weight of about 0.16 to about 0.36 gsm.. If too little nonionic surfactant is applied, the product will be damaged in the manufacturing process, especially during its axial ejection from the tampon press. If too much nonionic surfactant is applied, the excess tends to build up on the manufacturing equipment, and extraneous material (such as dirt, fibers, and the like) can become adhered to the machine. This can also result in poor control of the cover material, misplacement of the cover, and loose ends of the cover after cover attachment.

Again, the absorbent structure can be manufactured in a wide variety of sizes and shapes and from a wide variety of liquid-absorbing materials. An example of the formation of the absorbent structure is disclosed in Etz, US Pat. No. 3,417,102.

The nonionic surfactant may be applied to the cover in any manner that ensures sufficiently uniform coating. Many such methods are known to those of ordinary skill in the art. A representative, non-limiting list of useful methods, includes spraying, slot coating, brushing, transfer coating. The apertured film cover material may be applied to an absorbent structure in the manufacture of a tampon using a cut-and-place unit to cut the material from the slit roll and to place it on the absorbent structure. Another method of applying the cover is generally described in Friese, U.S. Patent No. 4,816,100. While this describes the use of a nonwoven cover to a tampon, improvements necessary to achieve this are described in the WO 01/02144 A1, entitled "Continuous Method of Providing Individual Sheets from a Continuous Web". This copending application discloses a method to achieve the total separation of a section of material comprises the following steps: severing a supply material in a plurality of discrete regions along a transverse axis, scoring the material residing between the severed regions along the same transverse axis, and then applying a force sufficient to fracture the scored regions, thereby separating the section of material from its supply.

The covered tampon blank can then be formed into a tampon using a tampon press. One method is described in Fig. 2A-2C. In this method, a tampon blank 200a comprising the absorbent structure substantially enclosed by the apertured film cover is compressed by the movement of a plurality of jaw members 210 and 212 in a tampon press 214. This procedure is described in detail in Wolff et al., US Pat. No. 3,422,496.

There are two distinct types of jaw members 210 and 212. The pointed jaws 210 serve to radially compress the tampon blank 200 along peripheral contact 216 lines lying on radial planes symmetrically disposed about a predetermined longitudinal press axis X, representing the central longitudinal axis intended for the compressed tampon 200c. The concave jaws 212 are constructed with concave cylindrical segment surface pressing portions 218. The jaws 210 and 212 are disposed for reciprocal movement radial paths converging to the central longitudinal axis X. The pointed jaws 210 and concave jaws 212 are arranged in a circumferential alternating sequence around the press axis X.

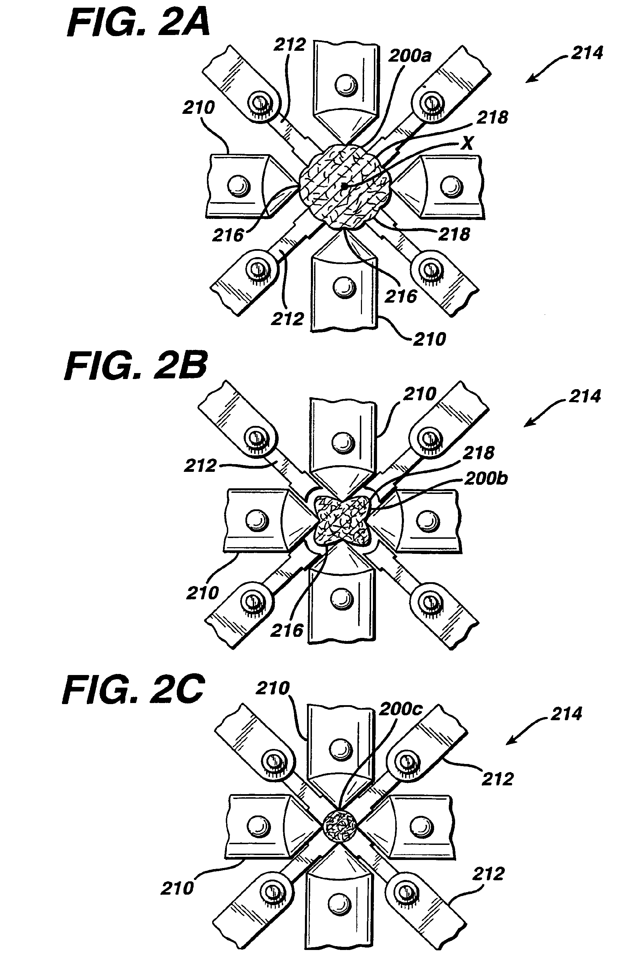

To compress the tampon blank 200a, it is placed in the center of the tampon press 214 with the central longitudinal axis of the tampon blank 200a substantially corresponding to the press axis X. The tampon blank 200a may be centered by the partial movement of all jaw members 210 and 212 as shown in Fig. 2a. The pointed jaws 210 move radially inwards toward the press axis X to a radial distance from the press axis X substantially corresponding to the radius of the compressed tampon 200c. The result of this movement is shown in Fig. 2b. Next, the concave jaws 212 move radially inwards to a radial distance from the press axis X substantially corresponding to the radius of the compressed tampon 200c as shown in Fig. 2c. Finally, the compressed tampon 200c is expelled out of the press (e.g., out of the plane of Figs 2A-2C) along the direction of the press axis X by means of a ram (not shown). This expulsion is axial or along the direction of the press axis, and it can be seen that expulsion forces can be quite high due to frictional forces between the compressed tampon 200c and the jaw members 210 and 212. While it may be possible to reduce the frictional forces by withdrawing the jaw members 210 and 212 slightly away from the press axis X, the radial expulsion of the compressed tampon 200c still provides significantly a high expulsion force.

Another method is described in Fig. 3A-3C. In this method, a tampon blank 200 comprising the absorbent structure substantially enclosed by the apertured film cover is compressed by the movement of a plurality of jaw members 210' and 212' in a tampon press 214'. This procedure is described in detail in Friese et al. US 2002/0157222 A1, and EP-B-0 422 660.

The jaw members 210 and 212 of Figs. 2A-2C are modified in this method to incorporate press cutters or fingers 220. The press cutters 220 serve to radially compress the core 22 of the tampon blank 200 along peripheral contact strips 224 lying on radial planes symmetrically disposed about a predetermined longitudinal press axis X, representing the central longitudinal axis intended for the compressed tampon 200. The jaw members 210' and 212' are disposed for reciprocal movement radial paths converging to the central longitudinal axis X. The jaw memberss 210' and 212' are arranged in a circumferential alternating sequence around the press axis X.

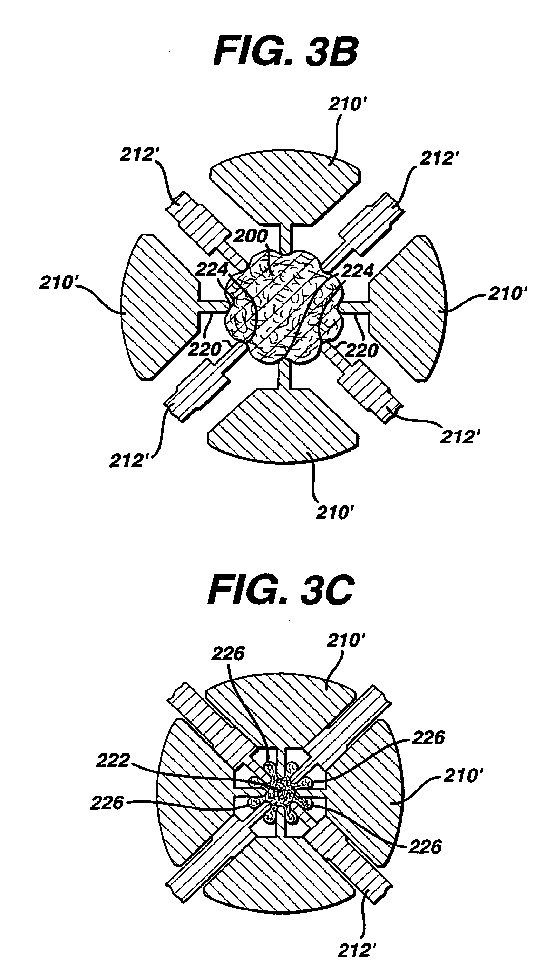

To compress the tampon blank 200, it is placed in the center of the tampon press 214' with the central longitudinal axis of the tampon blank 200 substantially corresponding to the press axis X. The tampon blank 200a may again be centered by the partial movement of all jaw members 210' and 212'. The jaw members 210' and 212' may move as described above for Figs. 2A-2C, or they may all move radially inwards toward the press axis X simultaneously. This latter movement is shown in Figs. 3B and 3C. In contrast to the method of Figs. 2A-2C, the distal ends of the press cutters 220 extend into the compressed tampon 200 between adjacent ribs 226. Finally, the compressed tampon 200 is expelled out of the press (as shown in Fig. 3A) along the direction of the press axis X by means of a ram 228. The outer portion of the ribs of the expelled tampon 200 may be further smoothed by means of a reducing bushing 230. This expulsion is axial or along the direction of the press axis, and it can be seen that expulsion forces can be quite high due to frictional forces between the compressed tampon 200 and the jaw members 210' and 212'. While it may be possible to reduce the frictional forces by withdrawing the jaw members 210' and 212' slightly away from the press axis X, the radial expulsion of the compressed tampon 200 still provides significantly a high expulsion force. This is especially true with the increased surface area of contact between the press cutters 220 and the compressed tampon 200.

Of course, one of ordinary skill in the art would recognize that it is helpful to use highly polished tools in the tampon presses of Figs. 2 and 3. In addition, it may be beneficial to treat the press surfaces with appropriate friction-reducing coatings.

### Examples

### Example 1

A series of trials was run to determine the effect of nonionic surfactant coatings of apertured film covers on tampon press ejection forces. Two polymeric blends were coextruded through multiple extruders. The two melt streams entered a feed block that split the outside layer polymer blend into two streams, leaving the intermediate layer intact. The outer layers thus enclosed or "sandwiched" the intermediate layer to produce an A-B-A film. 50 wt-% of the film was an immiscible blend of polypropylene and low density polyethylene ("LDPE") evenly distributed in the two A layers, and 50 wt-% of the film was an immiscible blend of linear low density polyethylene ("LLDPE") and ethylene-vinyl acetate copolymer (8 wt-% vinyl acetate) in the intermediate B layer. The A layers were formed of 70 wt-% of the polypropylene in the continuous phase and 30 wt-% of the LDPE in the dispersed phase. This film was provided as P18-3964 by Clopay Plastic Products Company, Inc., of Cincinnati, Ohio, USA, and it had an average basis weight of 20 gsm, a nominal thickness of 8 mils (0.2 mm), and a titanium dioxide pigment loading of about 6.5 wt-%.

The A-B-A film was then apertured by applying jets of hot air and vacuum at about 330 C while being supported by a cylindrical forming surface substantially as described in James et al., US Pat. No. 5,916,462, and Zimmerli, US Pat. No. 3,054,148. The differences between the disclosures therein and the process used herein would not be expected to change the results described hereinbelow. The resulting apertured film had a repeating pattern of substantially uniform, round apertures having a diameter of about 0.75 mm, an open area of about 32%, and an equivalent hydraulic diameter ("EHD"), as measured by the formula EHD = 4 * area/perimeter, of about 26 mils (0.65 mm).

Open area may be determined by using image analysis to measure the relative percentages of apertured and unapertured, or land, areas. Essentially image analysis converts an optical image from a light microscope into an electronic signal suitable for processing. An electronic beam scans the image, line-by-line. As each line is scanned, an output signal changes according to illumination. White areas produce a relatively high voltage and black areas a relatively low voltage. An image of the apertured formed film is produced and, in that image, the holes are white, while the solid areas of thermoplastic material are at various levels of gray. The more dense the solid area, the darker the gray area produced. Each line of the image that is measured is divided into sampling points or pixels. The following equipment can be used to carry out the analysis described above: a Quantimet Q520 Image Analyzer (with v. 5.02B software and Grey Store Option), sold by LEICA/Cambridge Instruments Ltd., in conjunction with an Olympus SZH Microscope with a transmitted light base, a plan 1.0x objective, and a 2.50x eyepiece. The image can be produced with a DAGE MTI CCD72 video camera.

A representative piece of each material to be analyzed is placed on the microscope stage and sharply imaged on the video screen at a microscope zoom setting of 10x. the open area is determined from field measurements of representative areas. The Quantimet program output reports mean value and standard deviation for each sample.

EHD was measured according to the procedure disclosed in Turi et al., US Pat. No. 5,567,376. However, the image was acquired using a ScanJet 4c scanner from Hewlett-Packard, Palo Alto, California, USA, and analyzed using Image-Pro software from Media Cybernetics, Silver Springs, Maryland, USA. These changes do not significantly alter any results.

The apertured film was coated with polysorbate 20 (TWEEN 20, available from ICI, Atlas Chemical Division, of Wilmington, Delaware, USA, by applying a fine spray of a solution formed of 1 part TWEEN 20 dissolved in 2 parts (v/v) isopropyl alcohol, and it was slit to a width of about 47 mm. TWEEN 20 was applied to the film substrate at ambient temperature of about 20 C at the target coating weights listed below in Table 1. The coating weight is measured based upon the TWEEN 20 as the alcohol solvent volatilizes.

The slit film was cut to form a cover having a length of about 125 mm. The cover is applied to an absorbent web comprising 75 wt-% rayon and 25 wt-% cotton having a length of about 235 mm, a width of about 50 mm and a target weight of about 2.5 g. The cover was heat sealed to one end of the absorbent web in a manner generally described in Friese, US Pat. No. 4,816,100, and copending application WO 01/02144 A1, entitled "Continuous Method of Providing Individual Sheets from a Continuous Web". The covered web was then compressed in a tampon press, as generally described in Friese et al., US 2002/0157222 A1, and EP-B-0 422 660.

The resulting tampons had a weight of between 2.55 and 3.2 g. The ejection ram of this press was fitted with a strain gauge to determine the ejection force required to eject the compressed tampon out of the press and through the reducing bushing. The ejection force was also recorded in Table 1.

**Table 1**

| Add-on Category* | Average Force Peak (N) | Comments |
|---|---|---|
| Low | 1155 | |
| Low | 1104 | |
| Low | 1110 | Some samples were below 2.55g. |
| Low | 1236 | |
| Target | 951 | |
| Target | 858 | |
| Target | 963 | |
| Target | 955 | |
| Target | 909 | |
| Target | 953 | |
| None | 1764 | For short periods of time. Press jammed. |
| None | 1799 | For short periods of time. Press jammed. |

| | | |
|---|---|---|
| *Add-on Category defined as: Low, 0.3-0.6 wt-%; Target, about 1 wt-%. | | |

The trial illustrates that it was possible to form tampons using an average ejection force of about 1200 N, or less. This was made possible by applying at least 0.3 wt-% TWEEN-20 to the cover material. However, about 1 wt-% provided a lower ejection force. Without any TWEEN-20, the ejection forces were measured as high as about 1800N before the products jammed in the press. Addition of low coating weights of TWEEN-20 provided an ejection force of about 60-70% of that measured for the uncoated product. Addition of target coating weights of TWEEN-20 provided an ejection force of about 50-55% of that measured for the uncoated product. These reductions are significant.

### Example 2:

A second trial was run to determine the effect of other nonionic surfactants. The samples were prepared as above for Example 1 with the following exceptions: various nonionic surfactants were applied as shown below in Table 2; the coating level was maintained at a target of 1 wt-%, and the coating was applied by kiss-coating. The line speed was varied while holding kiss roller speed at 18.4 ft/minute (5.6 m/min.) to provide the desired add-on level. While the coating temperature was varied to allow the nonionic surfactant to flow appropriately onto the substrate, ambient conditions were 72°F (22°C and 65% relative humidity. The nonionic surfactant, add-on level, and ejection forces are shown below in Table 2. Solid GML and BRIJ-76 (Steareth 10) were heated separately overnight in an oven at 150°F (65°C) to melt them, and they were applied from a Kiss Roller Pan, maintained at 150°F (65°C). The TWEEN-20 was applied from a Kiss Roller Pan, maintained at about 95°F (35°C).

**Table 2**

| Sample Size | Add-on Category* | Average Force Peak (N) | Std. Deviation (N) |
|---|---|---|---|
| 20 | 1.3 wt-% Brij 76 | 758 | 113 |
| 13 | 1.1 wt-% GML | 778 | 97 |
| 4 | 1.0 wt-% TWEEN 20 | 775 | 64 |

The trial illustrates that other nonionic surfactants were also acceptable as the coating for the apertured film cover. All of the nonionic surfactants processed at about 800 N or less. This represents an ejection force of less than about 50% of the uncoated tampons of Example 1. Again, these reductions are significant.

The specification and embodiments above are presented to aid in the complete and non-limiting understanding of the invention disclosed herein. Since many variations and embodiments of the invention can be made without departing from its scope, the invention resides in the claims hereinafter appended.

## Claims

1. A tampon (10, 200, 200c) comprising an absorbent structure (14) enclosed by a cover (12) wherein the cover has a nonionic surfactant coating applied thereto, the coating weight being in the range from 0.1 to 0.5 gsm, and comprises fluid-impervious plastic material in the form of a resilient three-dimensional web exhibiting a fiber-like appearance and tactile impression, the web has first and second surfaces, the first surface having a multiplicity of apertures therein, wherein
the fluid-impervious plastic material comprises a blend of at least two thermoplastic polymeric components, a continuous phase of a first thermoplastic polymeric component that exhibits a first melting point temperature and a dispersed phase of an immiscible, second thermoplastic polymeric component that exhibits a second melting point temperature, less than the first melting point temperature, such that when the web is heated to a temperature between the first melting point temperature and the second melting point temperature, the second thermoplastic polymeric component is capable of forming an adhesive bond, and wherein the cover is thermally bonded to the absorbent structure.

2. The tampon (10, 200, 200c) according to claim 1 wherein
the first thermoplastic polymeric component is present in the range from 45 to 95 wt-% and the second thermoplastic component is present in the range from 5 to 55 wt-% of the layer.

3. The tampon (10, 200, 200c) according to one of the preceding claims wherein
the fluid-impervious plastic material comprises a multilayer A-B-A film wherein the outer A layers comprise 70 wt-% of polypropylene as the first thermoplastic polymeric component in the continuous phase and 30 wt-% of low density polyethylene as the second thermoplastic polymeric component in the dispersed phase, and wherein the intermediate B layer comprises an immiscible blend of linear low density polyethylene and ethylene-vinyl acetate copolymer having 8 wt-% vinyl acetate, wherein the film comprises 50 wt-% of the immiscible blend of polypropylene and low density polyethylene evenly distributed in the two A layers and 50 wt-% of the blend for the intermediate B layer.

4. The tampon (10, 200, 200c) of claim 1 wherein
the cover (12) conducts fluid to the absorbent structure (14) enclosed therein.

5. The tampon (10, 200, 200c) of claim 1 wherein
the nonionic surfactant is an ethoxylate.

6. The tampon (10, 200, 200c) of claim 5 wherein
the ethoxylate is selected from the group consisting of ethoxylated fatty acids, polyolesters, polyoxyethylene alkyl esters and polyethylene glycol.

7. The tampon (10, 200, 200c) of claim 1 wherein
the first thermoplastic polymeric component is selected from the group consisting of polyolefins, polyesters, polyamides, polyurethanes, polystyrenes, halogenated polymers, and copolymers thereof.

8. The tampon (10, 200, 200c) of claim 1 wherein
the first thermoplastic polymeric component comprises a polyolefin.

9. The tampon (10, 200, 200c) of claim 1 wherein
the second thermoplastic polymeric component is selected from the group consisting of polyolefins, polyesters, polyamides, polyurethanes, polystyrenes, halogenated polymers, and copolymers thereof.

10. The tampon(10, 200, 200c) of claim 9 wherein
the second thermoplastic polymeric component comprises a polyolefin.

11. The tampon (10, 200, 200c) of claim 1 wherein
the difference between the first melting point temperature and the second melting point temperature is greater than 20° C.

12. The tampon (10, 200, 200c) of claim 1 which comprises
60 to 80 wt-% of the first thermoplastic polymeric component and 40 to 20 wt-% of the second thermoplastic polymeric component.

13. The tampon (10, 200, 200c) of claim 1 which further comprises
one or more components selected from the group comprising antioxidants, UV absobers, lubricants, antiblock agents, slip agents, plasticizers, nucleating agents, antistatic agents, flame retardants, pigments, dyes, and fillers.

14. A process for the manufacture of a tampon (10, 200, 200c) comprising:
a) coating a web (12) of fluid-impervious plastic material with a non-ionic surfactant, the coating weight being in the range from 0.1 to 0.5 gsm, the web (12) being in the form of resilient three-dimensional web exhibiting a fiber-like appearance and tactile impression, the web having opposed first and second edges and a substantially infinite length and first and a second surfaces, the first surface having a multiplicity of apertures therein, wherein
the fluid-impervious plastic material comprises a blend of at least two thermoplastic polymeric components, a continuous phase of a first thermoplastic polymeric component that exhibits a first melting point temperature and a dispersed phase of an immiscible, second thermoplastic polymeric component that exhibits a second melting point temperature, less than the first melting point temperature, such that when the web is heated to a temperature between the first melting melting point temperature and the second melting point temperature, the second thermoplastic polymeric component is capable of forming an adhesive bond,
b) separating an individual sheet from the web;
c) attaching the individual sheet to an absorbent sliver by applying heat and pressure to thermally bond the sheet to the absorbent sliver;
d) forming the absorbent sliver into a tampon blank wherein the individual sheet encloses the tampon blank;
e) compressing the tampon blank (200a) in a press (214) to form a cylindrical, compressed tampon (10, 200, 200c) having a cover (12) comprising the individual sheet; and
f) applying an ejection force to the compressed tampon (10, 200, 200c) in an axial direction to eject the tampon from the press (214), and wherein a sufficient amount of the surfactant is applied to reduce the ejection force to less than 80% compared with the ejection force of an uncoated tampon.

15. The process of claim 14 wherein 0.1 to 0.4 gsm of the non-ionic surfactant are applied as a coating.

16. The process of claim 14 wherein
the non-ionic surfactant is an ethoxylate.

17. The process of claim 16 wherein
the ethoxylate is selected from the group consisting of ethoxylated fatty acids, polyolesters, polyoxyethylene alkyl esters and polyethylene glycol.

18. The process of claim 14 wherein the non-ionic surfactant is applied in an amount sufficient to reduce the ejection force to less than 1100 N.

19. The process of claim 15 wherein
the non-ionic surfactant is applied at 0.16 to 0.38 gsm.

20. The process according to one of claims 14 to 19 wherein
the coating is conducted by spraying, slot coating, brushing or transfer coating.

## Patentansprüche

1. Tampon (10, 200, 200c), der eine absorbierende Struktur (14) umfaßt, die von einer Abdeckung (12) umhüllt ist, wobei die Abdeckung über eine auf dieser aufgebrachte Beschichtung mit einer nichtionischen grenzflächenaktiven Substanz verfügt, wobei das Beschichtungsgewicht im Bereich von 0,1 bis 0,5 g/m² liegt, und die ein flüssigkeitsundurchlässiges Kunststoffmaterial in Form eines elastischen dreidimensionalen Gewebes umfaßt, das ein faserähnliches Aussehen und ein faserähnliches Berührungsempfinden aufweist, wobei das Gewebe erste und zweite Oberflächen hat, die erste Oberfläche eine Vielzahl von Öffnungen in dieser aufweist, **dadurch gekennzeichnet, daß**
das flüssigkeitsundurchlässige Kunststoffmaterial umfaßt eine Mischung von zumindest zwei thermoplastischen polymeren Bestandteilen, eine kontinuierliche Phase eines ersten thermoplastischen polymeren Bestandteils, der eine erste Schmelzpunkttemperatur hat, und eine disperse Phase eines nicht-mischbaren, zweiten thermoplastischen polymeren Bestandteils, der eine zweite Schmelzpunkttemperatur hat, die niedriger als die erste Schmelzpunkttemperatur ist, dergestalt, daß, wenn das Gewebe auf eine Temperatur zwischen der ersten Schmelzpunkttemperatur und der zweiten Schmelzpunkttemperatur erwärmt wird, der zweite thermoplastische polymere Bestandteil in der Lage ist, eine Klebeverbindung auszubilden und wobei die Abdeckung mit der absorbierenden Struktur thermisch verbunden wird.

2. Tampon (10, 200, 200c) nach Anspruch 1, **dadurch gekennzeichnet, daß** der erste thermoplastische polymere Bestandteil im Bereich von 45 bis 95 Gew.-% und der zweite thermoplastische Bestandteil im Bereich von 5 bis 55 Gew.-% der Lage vorliegt.

3. Tampon (10, 200, 200c) nach einem der vorangehenden Ansprüche, **dadurch gekennzeichnet, daß** das flüssigkeitsundurchlässige Kunststoffmaterial eine mehrlagige A-B-A Folie umfaßt, wobei die äußeren Lagen A 70 Gew.-% an Polypropylen als ersten thermoplastischen polymeren Bestandteil in der kontinuierlichen Phase enthalten und 30 Gew.-% an Polyethylen niedriger Dichte als zweiten thermoplastischen polymeren Bestandteil in der dispersen Phase aufweisen, und wobei die Zwischenlage B eine unvermischbare Mischung eines Copolymers aus linearem Polyethylen niedriger Dichte und Ethylenvinylacetat mit 8 Gew.-% Vinylacetat aufweist, wobei die Folie 50 Gew.-% der nicht-mischbaren Mischung aus Polypropylen und Polyethylen niedriger Dichte, gleichmäßig in den zwei Schichten A verteilt, und 50 Gew.-% der Mischung für die Zwischenlage B aufweist.

4. Tampon (10, 200, 200c) nach Anspruch 1, **dadurch gekennzeichnet, daß** die Abdeckung (12) Flüssigkeit zu der in dieser eingeschlossenen absorbierenden Struktur (14) leitet.

5. Tampon (10, 200, 200c) nach Anspruch 1, **dadurch gekennzeichnet, daß** die nichtionische grenzflächenaktive Substanz ein Ethoxylat ist.

6. Tampon (10, 200, 200c) nach Anspruch 5, **dadurch gekennzeichnet, daß** das Ethoxylat aus der Gruppe, bestehend aus ethoxylierten Fettsäuren, Polyolestern, Polyoxyethylenalkylestern und Polyethylenglykol, ausgewählt wird.

7. Tampon (10, 200, 200c) nach Anspruch 1, **dadurch gekennzeichnet, daß** der erste thermoplastische polymere Bestandteil aus der Gruppe, bestehend aus Polyolefinen, Polyestern, Polyamiden, Polyurethanen, Polystyrolen, halogenierten Polymeren, sowie Copolymeren derselben, ausgewählt wird.

8. Tampon (10, 200, 200c) nach Anspruch 1, **dadurch gekennzeichnet, daß** der erste thermoplastische polymere Bestandteil ein Polyolefin umfaßt.

9. Tampon (10, 200, 200c) nach Anspruch 1, **dadurch gekennzeichnet, daß** der zweite thermoplastische polymere Bestandteil aus der Gruppe, bestehend aus Polyolefinen, Polyestern, Polyamiden, Polyurethanen, Polystyrolen, halogenierten Polymeren, sowie Copolymeren derselben, ausgewählt wird.

10. Tampon (10, 200, 200c) nach Anspruch 9, **dadurch gekennzeichnet, daß** der zweite thermoplastische polymere Bestandteil ein Polyolefin umfaßt.

11. Tampon (10, 200, 200c) nach Anspruch 1, **dadurch gekennzeichnet, daß** der Unterschied zwischen der ersten Schmelzpunkttemperatur und der zweiten Schmelzpunkttemperatur größer als 20° C ist.

12. Tampon (10, 200, 200c) nach Anspruch 1, der einen Massenanteil von 60 bis 80 Gew.-% des ersten thermoplastischen polymeren Bestandteils und einen Massenanteil von 40 bis 20 Gew.-% des zweiten thermoplastischen polymeren Bestandteils umfaßt.

13. Tampon (10, 200, 200c) nach Anspruch 1, der des weiteren einen oder mehr Bestandteile umfaßt, ausgewählt aus der Gruppe, die Antioxidantien, UV-absorbierende Mittel, Schmierstoffe, Anti-Blockierungsmittel, Gleitmittel, Weichmacher, Nukleirungsmittel, antistatische Mittel, Flammhemmer, Pigmente, Farbstoffe und Füllstoffe umfaßt.

14. Verfahren für die Herstellung eines Tampons (10, 200, 200c) umfassend:
a) Beschichtung eines Gewebes (12) aus flüssigkeitsundurchlässigem Kunststoffmaterial mit einer nicht-ionischen grenzflächenaktiven Substanz, wobei sich das Beschichtungsgewicht im Bereich von 0,1 bis 0,5 g/m² bewegt, das Gewebe (12) die Form eines elastischen dreidimensionalen Gewebes hat, das ein faserähnliches Aussehen und ein faserähnliches Berührungsempfinden aufweist, das Gewebe einander gegenüberliegende erste und zweite Ränder und eine im wesentlichen unendliche Länge und eine erste und eine zweite Oberfläche hat, wobei die erste Oberfläche eine Vielzahl von Öffnungen in dieser aufweist, **dadurch gekennzeichnet, daß** das flüssigkeitsundurchlässige Kunststoffmaterial umfaßt eine Mischung von zumindest zwei thermoplastischen polymeren Bestandteilen, eine kontinuierliche Phase eines ersten thermoplastischen polymeren Bestandteils, der eine erste Schmelzpunkttemperatur aufweist, und eine disperse Phase eines nicht-mischbaren, zweiten thermoplastischen polymeren Bestandteils, der eine zweite Schmelzpunkttemperatur aufweist, die niedriger ist als die erste Schmelzpunkttemperatur, so daß, wenn das Gewebe auf eine Temperatur zwischen der ersten Schmelzpunkttemperatur und der zweiten Schmelzpunkttemperatur erwärmt wird, der zweite thermoplastische polymere Bestandteil in der Lage ist, eine Klebeverbindung auszubilden,
b) Abtrennung einer einzelnen Lage vom Gewebe;
c) Befestigung der einzelnen Lage an einem absorbierenden Faserband durch das Aufbringen von Wärme und Druck, um die Lage mit dem absorbierenden Faserband thermisch zu verbinden;
d) Ausbildung des absorbierenden Faserbandes zu einem Tamponrohling, wobei die einzelne Lage den Tamponrohling umschließt;
e) Komprimieren des Tamponrohlings (200a) in einer Presse (214) für das Ausbilden eines zylindrischen, komprimierten Tampons (10, 200, 200c), der eine Abdeckung (12) aufweist, welche die einzelne Lage umfaßt; und
f) Ausüben einer Auswurfkraft auf den komprimierten Tampon (10, 200, 200c) in axialer Richtung, um den Tampon aus der Presse (214) auszuwerfen, wobei eine ausreichende Menge der grenzflächenaktiven Substanz aufgebracht wird, um die Auswurfkraft auf weniger als 80 % im Vergleich zur Auswurfkraft eines unbeschichteten Tampons zu reduzieren.

15. Verfahren nach Anspruch 14, **dadurch gekennzeichnet, daß** 0,1 bis 0,4 g/m² der nichtionischen grenzflächenaktiven Substanz als Beschichtung aufgebracht werden.

16. Verfahren nach Anspruch 14, **dadurch gekennzeichnet, daß** die nichtionische grenzflächenaktive Substanz ein Ethoxylat ist.

17. Verfahren nach Anspruch 16, **dadurch gekennzeichnet, daß** das Ethoxylat ausgewählt wird aus der Gruppe, bestehend aus ethoxylierten Fettsäuren, Polyolestern, Polyoxyethylenalkylestern und Polyethylenglykol.

18. Verfahren nach Anspruch 14, **dadurch gekennzeichnet, daß** die nichtionische grenzflächenaktive Substanz in einer Menge aufgebracht wird, die ausreicht, um die Auswurfkraft auf weniger als 1100 N zu reduzieren.

19. Verfahren nach Anspruch 15, **dadurch gekennzeichnet, daß** die nichtionische grenzflächenaktive Substanz mit 0,16 bis 0,38 g/m² aufgebracht wird.

20. Verfahren nach einem der Ansprüche 14 bis 19, **dadurch gekennzeichnet, daß** die Beschichtung durch Sprayen, Schlitzbeschichten, Aufbürst- oder Transferbeschichtung vorgenommen wird.

## Revendications

1. Tampon (10, 200, 200c) comprenant une structure absorbante (14) enfermée dans une pellicule (12), dans lequel la pellicule porte un revêtement de tensioactif non ionique appliqué sur celle-ci, le poids de revêtement étant dans la gamme de 0,1 à 0,5 g/m², et comprend une matière plastique imperméable aux fluides sous la forme d'une toile élastique tri-dimensionnelle présentant l'aspect d'une fibre et une impression tactile, la toile comprend une première et une deuxième surfaces, la première surface ayant une multiplicité d'ouvertures, dans lequel :
la matière plastique imperméable aux fluides comprend un mélange d'au moins deux composants polymères thermoplastiques, une phase continue d'un premier composant polymère thermoplastique qui présente une première température de point de fusion et une phase dispersée d'un deuxième composant polymère thermoplastique non miscible qui présente une deuxième température de point de fusion, inférieure à la première température de point de fusion, de sorte que lorsque la toile est chauffée à une température comprise entre la première température de point de fusion et la deuxième température de point de fusion, le deuxième composant polymère thermoplastique est capable de former une liaison adhésive, et dans lequel la pellicule est liée thermiquement à la structure absorbante.

2. Tampon (10, 200, 200c) selon la revendication 1, dans lequel
le premier composant polymère thermoplastique est présent dans une gamme de 45 à 95 % en poids et le deuxième composant thermoplastique est présent dans une gamme de 5 à 55 % en poids de la couche.

3. Tampon (10, 200, 200c) selon l'une des revendications précédentes, dans lequel
la matière plastique imperméable aux fluides comprend un film multicouche A-B-A dans lequel les couches extérieures A comprennent 70 % en poids de polypropylène en tant que premier composant polymère thermoplastique dans la phase continue et 30 % en poids de polyéthylène basse densité en tant que deuxième composant polymère thermoplastique dans la phase dispersée, et dans lequel la couche intermédiaire B comprend un mélange non miscible de polyéthylène linéaire basse densité et d'un copolymère éthylène - acétate de vinyle comprenant 8 % en poids d'acétate de vinyle, dans lequel le film comprend 50 % en poids du mélange non miscible de polypropylène et de polyéthylène basse densité réparti uniformément dans les deux couches A et 50 % en poids du mélange pour la couche intermédiaire B.

4. Tampon (10, 200, 200c) selon la revendication 1, dans lequel
la pellicule (12) conduit un fluide vers la structure absorbante (14) enfermée dans celle-ci.

5. Tampon (10, 200, 200c) selon la revendication 1, dans lequel
le tensioactif non ionique est un éthoxylate.

6. Tampon (10, 200, 200c) selon la revendication 5, dans lequel
l'éthoxylate est choisi dans le groupe constitué par les acides gras éthoxylés, les polyolesters, les esters alkyliques polyéthoxylés et le polyéthylèneglycol.

7. Tampon (10, 200, 200c) selon la revendication 1, dans lequel
le premier composant polymère thermoplastique est choisi dans le groupe constitué par les polyoléfines, les polyesters, les polyamides, les polyuréthanes, les polystyrènes, les polymères halogénés et les copolymères de ceux-ci.

8. Tampon (10, 200, 200c) selon la revendication 1, dans lequel
le premier composant polymère thermoplastique comprend une polyoléfine.

9. Tampon (10, 200, 200c) selon la revendication 1, dans lequel
le deuxième composant polymère thermoplastique est choisi dans le groupe constitué par les polyoléfines, les polyesters, les polyamides, les polyuréthanes, les polystyrènes, les polymères halogénés et les copolymères de ceux-ci.

10. Tampon (10, 200, 200c) selon la revendication 9, dans lequel
le deuxième composant polymère thermoplastique comprend une polyoléfine.

11. Tampon (10, 200, 200c) selon la revendication 1, dans lequel
la différence entre la première température de point de fusion et la deuxième température de point de fusion est supérieure à 20°C.

12. Tampon (10, 200, 200c) selon la revendication 1, qui comprend
60 à 80 % en poids du premier composant polymère thermoplastique et 40 à 20 % en poids du deuxième composants polymère thermoplastique.

13. Tampon (10, 200, 200c) selon la revendication 1, qui comprend en outre
un ou plusieurs composants choisis dans le groupe constitué par les antioxydants, les absorbeurs d'UV, les lubrifiants, les agents anti-blocage, les agents de glissement, les plastifiants, les agents de nucléation, les agents antistatiques, les retardateurs d'inflammation, les pigments, les colorants et les charges.

14. Procédé de fabrication d'un tampon (10, 200, 200c) comprenant :
a) le revêtement d'une toile (12) d'une matière plastique imperméable aux fluides avec un tensioactif non ionique, le poids de revêtement étant dans la gamme de 0,1 1 à 0,5 g/m², la toile (12) étant sous la forme d'une toile élastique tri-dimensionnelle présentant l'aspect d'une fibre et une impression tactile, la toile ayant un premier et un deuxième bords opposés et une longueur essentiellement infinie et une première et une deuxième surfaces, la première surface ayant une multiplicité d'ouvertures, dans lequel : la matière plastique imperméable aux fluides comprend un mélange d'au moins deux composants polymères thermoplastiques, une phase continue d'un premier composant polymère thermoplastique qui présente une première température de point de fusion et une phase dispersée d'un deuxième composant polymère thermoplastique non miscible qui présente une deuxième température de point de fusion, inférieure à la première température de point de fusion, de sorte que lorsque la toile est chauffée à une température comprise entre la première température de point de fusion et la deuxième température de point de fusion, le deuxième composant polymère thermoplastique est capable de former une liaison adhésive,
b) la séparation d'une feuille individuelle de la toile ;
c) la fixation de la feuille individuelle sur un ruban absorbant par application de chaleur et d'une pression pour lier thermiquement la feuille au ruban absorbant ;
d) la formation du ruban absorbant dans un tampon brut dans lequel la feuille individuelle enferme le tampon brut ;
e) la compression du tampon brut (200a) dans une presse (214) pour former un tampon cylindrique comprimé (10, 200, 200c) comprenant une pellicule (12) comprenant la feuille individuelle ; et
f) l'application d'une force d'éjection sur le tampon comprimé (10, 200, 200c) dans une direction axiale pour éjecter le tampon hors de la presse (214), et où une quantité suffisante de tensioactif est appliquée pour réduire la force d'éjection à moins de 80 % par rapport à la force d'éjection d'un tampon non revêtu.

15. Procédé selon la revendication 14, dans lequel 0,1 à 0,4 g/m² du tensioactif non ionique est appliqué sous forme d'un revêtement.

16. Procédé selon la revendication 14, dans lequel le tensioactif non ionique est un éthoxylate.

17. Procédé selon la revendication 16, dans lequel l'éthoxylate est choisi dans le groupe constitué par les acides gras éthoxylés, les polyolesters, les esters alkyliques polyéthoxylés et le polyéthylèneglycol.

18. Procédé selon la revendication 14, dans lequel le tensioactif non ionique est appliqué dans une quantité suffisante pour réduire la force d'éjection à moins de 1100 N.

19. Procédé selon la revendication 15, dans lequel le tensioactif non ionique est appliqué à raison de 0,16 à 0,38 g.

20. Procédé selon l'une des revendications 14 à 19, dans lequel le revêtement est réalisé par pulvérisation, revêtement à la fente, revêtement à la brosse ou par transfert.
